# EUROPEAN PATENT APPLICATION

(11) **EP 2 752 782 A2**
(43) Date of publication of application: **09.07.2014**
(21) Application number: 13198510.3
(22) Date of filing: 19.12.2013
(51) Int. Cl.: G06F 19/00

(54) **Physiological information monitoring system**

(30) Priority: 08.01.2013 JP 2013001238
(71) Applicant: Nihon Kohden Corporation, Shinjuku-ku Tokyo (JP)
(72) Inventor: Ikeya, Hirohiko, Shinjuku-ku, Tokyo (JP); Ukawa, Teiji, Shinjuku-ku, Tokyo (JP); Kawasaki, Nobuko, Shinjuku-ku, Tokyo (JP); Kawano, Junya, Shinjuku-ku, Tokyo (JP); Suzuki, Takaharu, Shinjuku-ku, Tokyo (JP)
(74) Representative: Grünecker, Kinkeldey, Stockmair & Schwanhäusser

(57) **Abstract**

A physiological information monitoring system for monitoring physiological information on a patient is provided. A first monitor has a first display and a first connector. A second portable monitor has a second display, a second connector, and a signal input unit. A measurement signal of physiological information is input to the signal input unit. The second display unit displays information corresponding to the measurement signal. When the first connector and the second connector are connected, the second display is brought into a non-display status, whereupon the first display displays information corresponding to the measurement signal.

## Description

### BACKGROUND

The invention relates to a physiological information monitoring system for monitoring physiological information on a patient, or the like.

USP4,688,579 discloses that an signal input device is plugged into a slot defined in a monitor having a display of a patient monitoring system. Such a configuration enables elimination of troublesome work for detaching electrodes attached to a patient and attaching a cable to another monitor when the patient, or the like, is required to move apart from the monitor.

[Patent Document 1] Japanese Utility Model Registration No. 1981633

In the configuration described in USP4,688,579, a removably attachable signal input device itself does not have a display. Accordingly, measurement results of physiological information on the patient who has moved apart from the monitor are stored in memory provided in the signal input device. In order to visually check the thus-stored measurement results, the signal input device must be plugged into the monitor again to thereby display the measurement results on the display. Consequently, in a state where the patient stays away from the monitor, the physiological information cannot be monitored in real time, difficulty is encountered in applying the monitoring system to; for instance, a patient whose health condition may suddenly turn for the worse.

### SUMMARY

This invention provides a technique for enabling real-time monitoring of physiological information even when the patient stays apart from the monitor.

An aspect of the invention provides a physiological information monitoring system comprising: a first monitor including a first display and a first connector; and a second monitor including a second connector removably attachable to the first connector, a signal input unit into which a measurement signal of physiological information is input, and a second display that displays information corresponding to the measurement signal, the second display being brought into a non-display status when the first connector and the second connector are connected to each other, and the information corresponding to the measurement signal are displayed on the first display.

In such a configuration, the second monitor configured as being removably attachable to the first monitor has a second display capable of displaying physiological information on a patient, in real time. Hence, the physiological information can be visually monitored in real time without undergoing locational constraints. Even when a health condition of the patient has suddenly turned for the worse, details of the physiological information can be visually checked, so that appropriate medical actions can be taken promptly.

In addition, when the first connector and the second connector are connected to each other, the second display is brought into a non-display status, and information corresponding to a measurement signal is displayed on the first display. Accordingly, redundantly displaying identical physiological information on both the first display and the second display is avoided, and unwanted power consumption can be curbed. Further, a heat buildup in the second monitor can be curbed, so that measures against the heat buildup in the first monitor to which the second monitor is to be attached can be simplified, which enables miniaturization of the system and curtailing of parts costs.

In a case where the first monitor is detected as being inactive when the first connector and the second connector are connected to each other, the second monitor may generate an alarm before bringing the second display into a non-display status.

Such a configuration makes it possible to avoid interruption of continual monitoring of physiological information, which would otherwise arise when the second display is brought into a non-display status while the first monitor remains inactive.

The first display unit and the second display unit may be arranged so as to face in different directions when the first connector and the second connector are connected.

In this case, an open space of the first monitor can be effectively utilized. Specifically, since a part of a housing of the first monitor where the first display is not provided has a large open space, an attachment area for the second monitor can be widely assured.

An indentation including the first connector may be formed in a portion of the first monitor; the second connector may be connected to the first connector as a result of the second monitor being put in the indentation; and a portion of an exterior surface of the second monitor may be flush with a portion of an exterior surface of the first monitor.

Such a configuration enables miniaturization of the entire monitoring system by effective utilization of an open space in the housing of the first monitor as the attachment area for the second monitor, which contributes to assuring a wide bedside open space. A region of the first monitor from which the attached second monitor projects is made small, thereby circumventing a likelihood that a body or cloth of the patient, and a healthcare personnel will be inadvertently caught by the system.

The second display may be capable of temporally displaying on the second display information which is being displayed on the first display under the condition that the first connector is connected to the second connector.

Such a configuration enables fulfillment of a request to temporarily check physiological information from a position different from the position where the first display is disposed. In addition, power consumption and a heat buildup can be minimized due to the fact that the duration of display time is limited.

### BRIEF DESCRIPTION OF THE DRAWINGS

Figs. 1A and 1B are block diagrams illustrating a monitoring system of an embodiment of the invention;
Fig. 2 is a perspective view illustrating a state in which a first monitor and a second monitor, which make up the monitoring system, are connected to each other;
Fig. 3 is a perspective view illustrating a state in which the first monitor and the second monitor are separated from each other;
Fig. 4 is a flowchart illustrating power management processing to be performed by the second monitor; and
Fig. 5 is a perspective view illustrating a state in which the first monitor and the second monitor, which belong to a modification, are separated from each other.

### DETAILED DESCRIPTION OF EMBODIMENTS

An embodiment of the invention is hereunder described in detail by reference to the accompanying drawings. Throughout the drawings employed in the following descriptions, a scaling factor is changed as appropriate in order to make respective members recognizable.

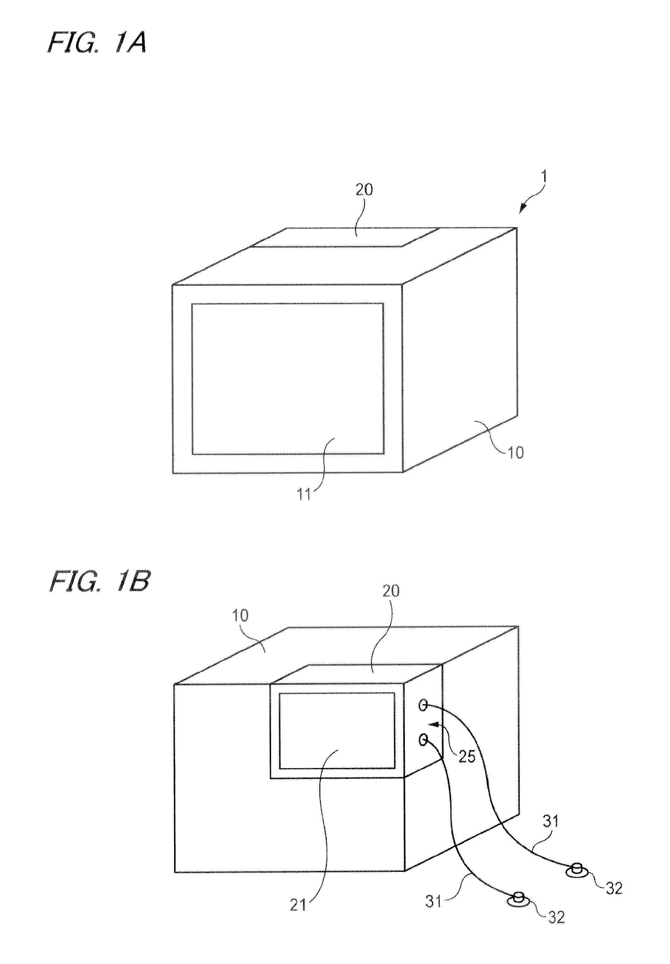

Figs. 1A and 1B are block diagrams illustrating a configuration of a monitoring system 1 according to an embodiment of the invention. The monitoring system 1 may include a first monitor 10 and a second monitor 20. Fig. 1A illustrates appearance of the first monitor 10 when viewed from its front, and Fig. 1B illustrates external appearance of the first monitor 10 when viewed from its rear.

The first monitor 10 is a bedside monitor, and an apparatus disposed beside a bed where a patient lies down in a health care facility, or the like. A first display 11 is on a front of the first monitor 10. The first display 11 is a known display.

The second monitor 20 is attached to a back of the first monitor 10. In an attached state, a front of the second monitor 20 is oriented toward the back of the first monitor 10. A second display 21 is placed on the front of the second monitor 20. The second display 21 is a known display that has a screen which is smaller than that of the first display 11.

The second monitor 20 may include a signal input unit 25. A cable 31 is connected to the signal input unit 25, and a sensor 32 that acquires physiological information when attached to a body of the patient, or the like, is connected to a leading edge of the cable 31. The sensor 32 may include an electrode for acquiring an electrocardiographic waveform, a probe for acquiring arterial oxygen saturation and a heat rate, a cuff for acquiring blood pressure, and others.

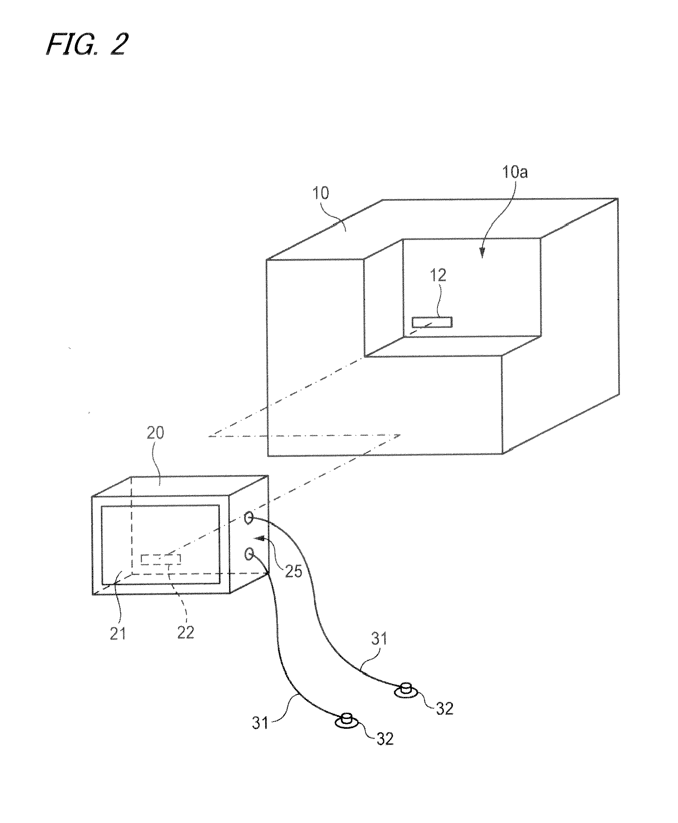

As shown in Fig. 2, the second monitor 20 is portable after being decoupled from the first monitor 10. The first monitor 10 has a first connector 12, and the second monitor 20 has a second connector 22. The second monitor 20 is attached to the first monitor 10 by coupling the first connector 10 to the second connector 20. Also, the second monitor 20 becomes detachable from the first monitor 10 by disconnecting the first connector 12 from the second connector 22.

For instance, when the patient, is transported from the bed, the second monitor 20 is detached from the first monitor 10 and fastened to another bed used in carrying the patient. Physiological information on the patient can be continuously monitored without detaching the sensor 32 put on the patient's body or disconnecting the cable 31 from the signal input unit 25.

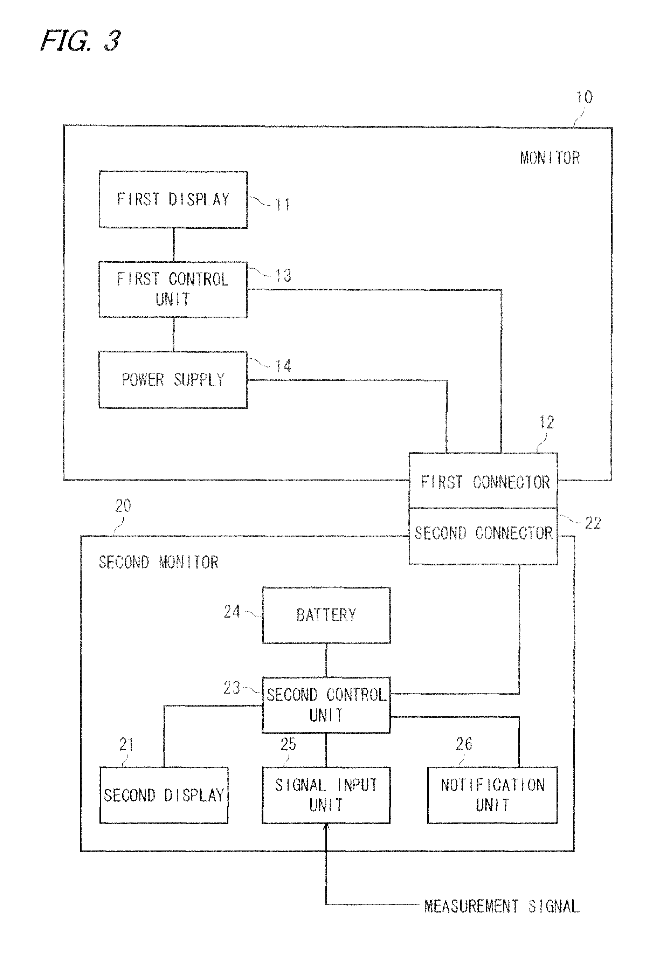

Fig. 3 is a block diagram illustrating a configuration of the monitoring system 1. In addition to including the first display 11 and the first connector 12, the first monitor 10 may further include a first control unit 13 and a power supply 14. In addition to including the second display 21, the second connector 22, and the signal input unit 25, the second monitor 20 may further include a second control unit 23, a battery 24, and a notification unit 26.

The first control unit 13 may include a CPU that performs various arithmetic processing operations, ROM that stores various control programs, RAM utilized as a work area for storing information and executing a program, or the like, and performs varieties of control operations of the first monitor 10. The first control unit 13 is connected so as to be able to communicate with the first display 11, the first connector 12, and the power supply 14.

The power supply 14 is configured so as to be connectable with commercial power. When an unillustrated power switch provided on the first monitor 10 is turned on, the first control unit 13 feeds electric power supplied from the commercial power to respective portions of the first monitor unit 10 by way of the power supply 14. The power supply 14 is electrically connected to the first connector 12.

The second control unit 23 may include a CPU that performs various arithmetic processing operations, ROM that stores various control programs, RAM utilized as a work area for storing information and executing a program, or the like, and performs varieties of control operations of the second monitor 20. The second control unit 23 is connected so as to be able to communicate with the second display 21, the second connector 22, the battery 24, the signal input unit 25, and the notification unit 26.

The battery 24 is a rechargeable battery having a known configuration. When an unillustrated power switch provided on the second monitor 20 is turned on, the second control unit 23 feeds electric power to respective portions of the second monitor 20 from the battery 24.

As mentioned previously, a measurement signal of physiological information on the patient, are input to the signal input unit 25 through the sensor 32 and the cable 31. The second control unit 23 displays information (a waveform, a numerical value, an index, or the like) corresponding to the measurement signal on the second display 21 in real time.

Since the second portable monitor 20 may include the second display 21 capable of displaying physiological information on the patient, in real time, the physiological information can be monitored by a visual check without undergoing locational constraints. Even when the patient's health condition has suddenly turned for the worse, details of physiological information can be checked by a visual inspection, so that appropriate measures can be promptly taken.

When the patient, returns to the bed, the second monitor 20 is coupled to the first monitor 10 again. The second control unit 23 is configured so as to transmit a measurement signal input from the signal input unit 25 to the first monitor 10 when the first connector 12 and the second connector 22 are connected together.

Specifically, the measurement signal is input to the first control unit 13 through the first connector 12 and the second connector 22. The first control unit 13 displays in real time data (a waveform, a numerical value, an index, and others) corresponding to the measurement signal on the first display 11 in real time. In the meantime, the second control unit 23 puts the second display 21 in a non-display status, thereby aborting a real-time display of the data corresponding to the measurement signal input from the signal input unit 25.

The power supply 14 of the first monitor 10 and the battery 24 of the second monitor 20 are electrically connected to each other through the first connector 12 and the second connector 22, whereby recharging of the battery 24 is commenced by means of electric power fed by the power supply 14.

In the monitoring system 1 of the embodiment, redundantly displaying identical physiological information on both the first display 11 and the second display 21 is avoided, thereby fulfilling a request for power conservation performance which curbing unwanted power consumption.

Since the second display 21 is brought into a non-display status, heat buildup in the second monitor 20 can be inhibited. Therefore, measurements against the heat buildup can be simplified in the first monitor 10 around the portion where the second monitor 20 is to be attached, which makes it possible to downsize the system and reduce parts costs.

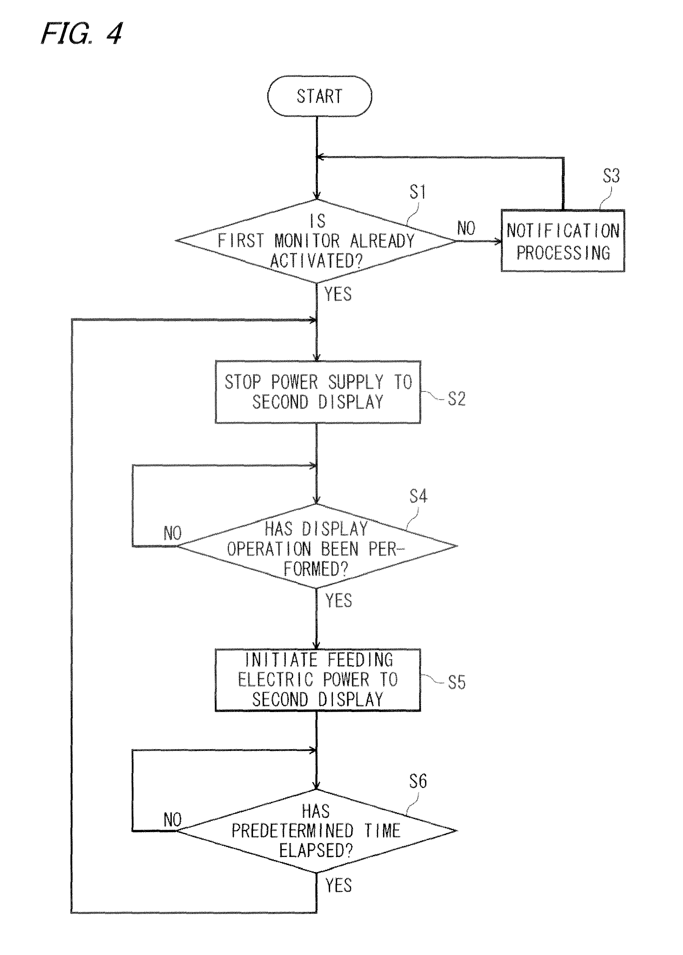

Power management processing to be performed by the second control unit 23 is now described more specifically by reference to a flowchart illustrated in Fig. 4. This power management processing may be stopped when the second control unit 23 detects that the first connector 12 and the second connector 22 are disconnected.

The second control unit 23 detects a coupling between the first connector 12 and the second connector 22, whereupon power management processing is initiated.

In accordance with a status of the first control unit 13 detected by way of the first connector 12 and the second connector 22, the second control unit 23 determines whether or not the first monitor 10 is already activated (step S1). The reason for this is that, if the second display 21 is brought into a non-display status while the first monitor 10 is inactive, continuity of physiological information monitoring will be unfavorably interrupted.

If the first monitor 10 is determined to be already activated (Yes in step S1), the second control unit 23 stops a power supply from the battery 24 to the second display 21, and the second display 21 is brought into a non-display status as mentioned above (step S2).

In the meantime, if the first monitor 10 is determined to be inactive (No in step S1), the second control unit 23 performs notification processing (step S3). To be specific, the notification unit 26 provided in the second monitor 20 generates an alarm, thereby notifying the user that the system is in an inappropriate use status. Notification is carried out by generating at least a visual alarm or an audible alarm.

Specifically, when the second monitor 20 detects that the first monitor 10 is inactive when the first connector 12 and the second connector 22 are connected, the second monitor 20 generates an alarm by the notification unit 26 before putting the second display 21 into a non-display status. Notification generating the alarm is continuously performed until activation of the first monitor 10 is detected. Continuity of physiological information monitoring can thereby be maintained. There may also be adopted another configuration for stopping the notification generating the alarm when the first connector 12 and the second connector 22 are disconnected.

Even when attached to the first monitor 10, the second monitor 20 of the embodiment can temporarily display on the second display 21 the physiological information that are on the first display 11, by performing predetermined operation. Moreover, system information on the first display 11, like remaining battery capacity, can be temporarily displayed on the second display 21. In this regard, however, in order to minimize power consumption and a heat buildup, it is desirable to limit a display time to a given value.

The second control unit 23 stays in a standby condition before predetermined display operation is performed (No in step S4). If predetermined display operation is detected (Yes in step S4), the second control unit 23 initiates feeding electric power from the battery 24 to the second display unit 21 (step S5), thereby letting the second display 21 display physiological information on the first display 11; namely, information corresponding to the measurement signal input to the signal input unit 25.

In the meantime, the second control unit 23 commences counting a predetermined display time by use of an internal timer. The counting is continued before a predetermined time elapses (No in step S6). If the counting the predetermined time ends (Yes in step S6), the second control unit 23 stops feeding the electric power from the battery 24 to the second display 21, whereupon the second display 21 is bought into a non-display status (step S2).

In the monitoring system 1 of the embodiment, the first display 11 and the second display 21 face in opposite directions when the second monitor 20 is coupled to the first monitor 10. Therefore, the above-mentioned function is useful for temporarily checking physiological information from a position opposite to the location where the first display 11 is placed.

The first display 11 can also be configured so as to display physiological information in real time, and the second display 21 can also be configured so as to display measurement results of physiological information stored in the past in memory of the first control unit 13 or the second control unit 23.

The first display 11 and the second display 21 are configured so as to face in opposite directions when the first connector 12 and the second connector 22 are connected to each other, thereby enabling effective utilization of an open space of the first monitor 10. To be specific, since a back side of a housing of the first monitor 10 where the first display 11 is not disposed has a large open space, an attachment area for the second monitor 20 can be assured widely.

In the embodiment, as shown in Fig. 2, an indentation 10a is formed in a part of the first monitor 10, and the first connector 12 is placed in the indentation 10a. The second connector 22 is placed on a back of the second monitor 20. As a result of the second monitor 20 being placed in the indentation 10a, the second connector 22 is connected to the first connector 12. The second monitor 20 is arranged at this time such that a portion of an exterior surface of the second monitor 20 becomes flush with a portion of an exterior surface of the first monitor 10.

The configuration enables miniaturization of the entire monitoring system 1 by effective utilization of an open space in the housing of the first monitor 10 as the attachment area of the second monitor 20, which contributes to assuring a wide bedside space. Further, a region of the first monitor 10 from which the attached second monitor 20 projects is made smaller, thereby circumventing a likelihood that a body or cloth of the patient, the healthcare personnel will be inadvertently caught by the system.

While the preferred embodiments of the invention have been illustrated and described, it will be appreciated that various changes can be made therein without departing from the spirit and scope of the invention.

The second monitor 20 is not always required to be arranged such that the second display 21 faces the opposite side of the first display 11 when the first connector 12 and the second connector 22 are connected to each other. If a sufficient space can be assured, there may also be adopted another configuration in which the second monitor 20 will be attached to a top surface and a side surface of the first monitor 10 and that the first connector 12 and the second connector 22 will face in different directions in such a state. In this case, the layout of the first connector 12 and the second connector 22 is determined as appropriate such that the orientations of the first display 11 and the second display 12 are accomplished.

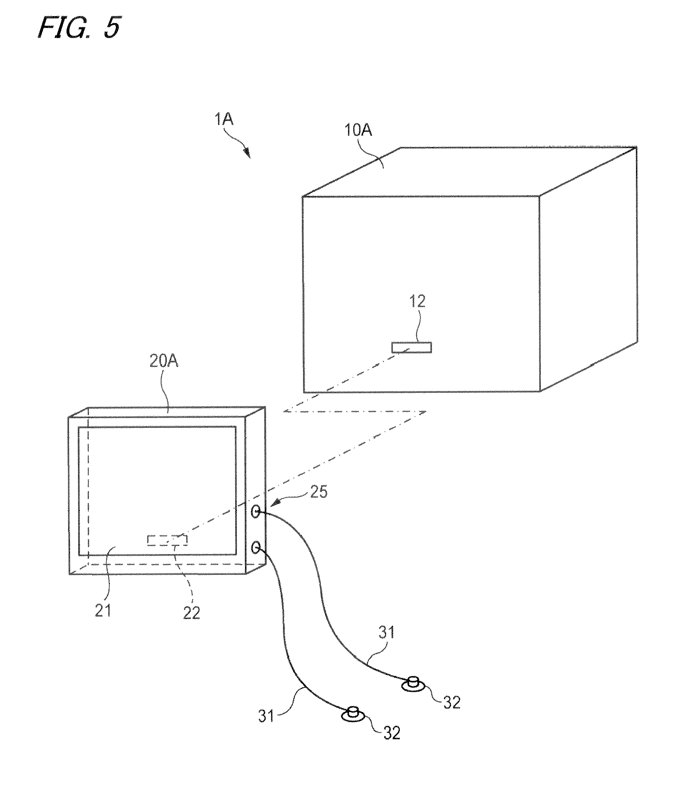

Further, the indentation 10a for accommodating the second monitor 20 does not always need to be formed in the portion of the first monitor 10. For instance, like a monitoring system 1A of a modification illustrated in Fig. 5, a tablet-shaped second monitor 20A can also be configured so as to be attached back-to-back to a first monitor 10A.

## Claims

1. A physiological information monitoring system comprising:
a first monitor including a first display and a first connector; and
a second monitor including a second connector removably attachable to the first connector, a signal input unit into which a measurement signal of physiological information is input, and a second display that displays information corresponding to the measurement signal,
the second display being brought into a non-display status when the first connector and the second connector are connected to each other, and the information corresponding to the measurement signal are displayed on the first display.

2. The physiological information monitoring system according to claim 1, wherein, in a case where the first monitor is detected as being inactive when the first connector and the second connector are connected to each other, the second monitor generates an alarm before bringing the second display into a non-display status.

3. The physiological information monitoring system according to claim 1 or 2, wherein the first display unit and the second display unit are arranged so as to face in different directions when the first connector and the second connector are connected.

4. The physiological information monitoring system according to any one of claims 1 to 3, wherein an indentation including the first connector is formed in a portion of the first monitor; the second connector is connected to the first connector as a result of the second monitor being put in the indentation; and a portion of an exterior surface of the second monitor is flush with a portion of an exterior surface of the first monitor.

5. The physiological information monitoring system according to any one of claims 2 to 4, wherein the second display is capable of temporally displaying on the second display information which is being displayed on the first display under the condition that the first connector is connected to the second connector.
